# EUROPÄISCHE PATENTSCHRIFT

(11) **EP 0 527 126 B1**
(45) Veröffentlichungstag und Bekanntmachung des Hinweises auf die Patenterteilung: **30.11.1994**
(21) Anmeldenummer: 90910103.2
(22) Anmeldetag: 10.07.1990
(51) Int. Cl.: G01N 27/416

(54) **ELEKTROCHEMISCHE BESTIMMUNG DER SAUERSTOFFKONZENTRATION**
ELECTROCHEMICAL DETERMINATION OF OXYGEN CONCENTRATION
DETERMINATION ELECTROCHIMIQUE DE LA CONCENTRATION EN OXYGENE

(30) Priorität: 02.05.1990 DE 4014109
(43) Veröffentlichungstag der Anmeldung: 17.02.1993
(73) Patentinhaber: Pacesetter AB, 171 95 Solna (SE)
(72) Erfinder: PREIDEL, Walter, D-8520 Erlangen (DE)
(74) Vertreter: Lettström, Richard Wilhelm
(86) Internationale Anmeldenummer: DE9000515
(87) Internationale Veröffentlichungsnummer: WO9117433

(56) Entgegenhaltungen:
- EP-A- 0 170 998

## Beschreibung

Die Erfindung betrifft ein Verfahren zur elektrochemischen Bestimmung der Sauerstoffkonzentration, insbesondere in Körperflüssigkeiten, mittels eines eine Sensorelektrode aufweisenden Sauerstoffsensors sowie einen nach diesem Verfahren arbeitenden Sauerstoffsensor.

Die Messung der Sauerstoffkonzentration bzw. des Sauerstoffpartialdruckes ist ein wichtiges analytisches Problem. Insbesondere in der medizinischen Technik ist eine rasche und genaue Ermittlung des Sauerstoffwertes notwendig. So erfordert die Bestimmung des Sauerstoffes im Blut von Patienten eine Genauigkeit von ca. 1 Torr, und zwar im Bereich zwischen 10 und 200 Torr. Die Drift des Signals sollte dabei im Verlauf von drei Tagen, was im wesentlichen der Dauer der Messung im Blut entspricht, einen Wert von 5 Torr nicht überschreiten. Die bislang in der Medizin verwendeten Sauerstoffsensoren genügen diesen Anforderungen aber bei weitem nicht.

In der medizinischen Technik wird derzeit im allgemeinen der Sauerstoffsensor nach Clark eingesetzt (siehe dazu: US-PS 2 913 386, 3 260 656 und 4 076 596), bei dem als Grundlage das elektrochemische Prinzip dient. Dieser Sensor kann nun zwar in Blutgasanalysatoren Verwendung finden, zur Bestimmung des Sauerstoffgehaltes im Blut ist er allerdings nicht geeignet, da einige wesentliche Sensormerkmale einer Implantation bzw. einem länger andauernden Betrieb im Körper entgegenstehen. Dazu zählt insbesondere eine hydrophobe Membran, die vor der Meßelektrode angeordnet ist. Diese Membran wird nämlich durch Wechselwirkungen im Körper in ihren Eigenschaften deutlich verändert. Außerdem führt der ständige und hohe Sauerstoffverbrauch des Sensors zu einer ausgeprägten Abstoßungsreaktion, die den Betrieb des Sensors weiter erschwert.

Aus der EP-OS 0 170 998 ist ein Verfahren zur elektrochemischen Bestimmung der Sauerstoffkonzentration, insbesondere in biologischem Material, mittels eines Sauerstoffsensors mit einer Meßelektrode und einer Gegenelektrode bekannt. Bei diesem Verfahren werden der Meßelektrode zyklisch zwei Potentiale aufgeprägt, wobei das eine Potential (Meßpotential) im Bereich zwischen -1,4 und -0,4 V liegt und das andere Potential (Erholungspotential) im Bereich zwischen -0,2 und +0,2 V, jeweils bezogen auf eine Ag/AgCl-Referenzelektrode; die Verweildauer beim Meßpotential ist dabei klein im Vergleich zur Zyklusdauer. Als Meßsignal wird bei diesem Verfahren der während der Meßpe riode fließende Strom ausgewertet, und zwar in Form des Ladungsintegrals. Das bekannte Verfahren dient in erster Linie dazu, relative Veränderungen der Sauerstoffkonzentration zu messen, um die Frequenz eines Herzschrittmachers dem Bedarf des Patienten anzupassen.

Aufgabe der Erfindung ist es, ein Verfahren anzugeben, mit welchem - unter Verwendung eines eine Sensorelektrode aufweisenden Sensors - eine exakte Sauerstoffbestimmung möglich ist, d.h. eine Bestimmung mit einer Genauigkeit von ca. 1 Torr.

Dies wird erfindungsgemäß dadurch erreicht, daß der Sensorelektrode ein Potentialprofil mit mehreren Potentialstufen aufgeprägt wird, wobei die erste Potentialstufe im Bereich zwischen -0,5 und -1,2 V (1. Meßpotential), die zweite Potentialstufe im Bereich zwischen -0,5 und -1,3 V (2. Meßpotential) und die dritte Potentialstufe etwa bei 0 V liegt, jeweils bezogen auf eine Ag/AgCl-Referenzelektrode, und wobei zwischen den beiden Meßpotentialen eine Potentialdifferenz von ≧ 50 mV besteht, daß die Verweildauer bei den beiden Meßpotentialen jeweils zwischen 10 und 50 ms beträgt (Meßperiode), daß der bei den beiden Meßpotentialen fließende Strom ermittelt und über die Zeit integriert wird, wobei die Integration jeweils etwa 5 bis 30 ms nach Beginn der Meßperiode einsetzt und jeweils zwischen 5 und 10 ms dauert, und daß durch Differenzbildung der beiden Integrale die Sauerstoffkonzentration ermittelt wird.

Beim erfindungsgemäßen Verfahren, bei dem der Sensorelektrode ein mehrere Potentialstufen umfassendes Potentialprofil zyklisch aufgeprägt wird und eine Auswertung der Stromantwort erfolgt, kann der Sauerstoffpartialdruck mit der geforderten Genauigkeit gemessen werden. Im einfachsten Fall besteht bei dieser Impulsmethode das Potentialprofil aus drei Potentialstufen, wobei der Strom während der beiden ersten Potentialstufen gemessen und jeweils das Integral über eine bestimmte Zeit ermittelt wird. Die Differenz der beiden Integrale ist dann eine Funktion der Sauerstoffkonzentration.

Der Meßwert ist beim erfindungsgemäßen Verfahren nur bei sehr geringen Flußgeschwindigkeiten (≈2 cm/s) vom Blutfluß abhängig. Dieses Verfahren eignet sich somit hervorragend für ein Monitoringsystem für den Sauerstoffpartialdruck im Blut; daneben kann es auch zur Sauerstoffbestimmung in Gewebsflüssigkeit dienen. Von Vorteil ist bei dieser Impulsmethode ferner der geringe Sauerstoffverbrauch; Körperreaktionen werden nämlich somit auf ein Mindestmaß reduziert.

Beim erfindungsgemäßen Verfahren, bei dem ein mehrfacher Potentialsprung erfolgt, wird mit zwei Meßpotentialen gearbeitet. Eines dieser beiden Meßpotentiale dient dabei zur Unterdrückung von Effekten, die unabhängig von der Sauerstoffkonzentration sind. Durch die Differenzbildung zwischen einem Integral (über dem Strom) bei einem Potential, bei dem die Sauerstoffreduktion weniger stark ausgeprägt ist, dafür aber der Nullstrom vorhanden ist, beispielsweise bei -0,8 V, und einem Integral (über dem Strom) bei einem Potential mit normal ausgeprägter Sauerstoffreduktion, beispielsweise bei -1,0 V, läßt sich nämlich der Nulleffekt unterdrücken. Der Vorteil besteht dabei darin, daß das Meßsignal bei einem Sauerstoffpartialdruck von 0 Torr etwa bei 0 As liegt. Infolgedessen ist die Eichung einfacher und stabiler, da auch ein Driften des Meßsignals zu Beginn des Sensorbetriebes verringert wird.

Das erste Meßpotential, d.h. der Potentialbereich des ersten Meßimpulses, liegt beim erfindungsgemäßen Verfahren im Bereich zwischen -0,5 und -1,2 V und vorteilhaft im Bereich zwischen -0,75 und -1,15 V; vorzugsweise beträgt das erste Meßpotential ca. -0,8 V. Das zweite Meßpotential, d.h. der Potentialbereich des zweiten Meßimpulses, liegt im Bereich zwischen -0,5 und -1,3 V und vorteilhaft im Bereich zwischen -0,85 und -1,25 V; vorzugsweise beträgt das zweite Meßpotential ca. -1,0 V. Die dritte Potentialstufe, d.h. die Potentialstufe nach den beiden Meßpotentialen, liegt etwa bei 0 V (mit einer Bandbreite von ca. ± 200 mV); die Verweildauer bei dieser Potentialstufe beträgt vorteilhaft zwischen 0,5 und 10 s. Die Potentialangaben beziehen sich dabei jeweils auf eine Ag/AgCl-Referenzelektrode.

Die Verweildauer bei den beiden Meßpotentialen, d.h. die zeitliche Länge der Meßimpulse, beträgt jeweils 10 bis 50 ms; vorzugsweise beträgt diese Zeitspanne jeweils ca. 20 ms. Die Integration des Stromes erfolgt jeweils etwa 5 bis 30 ms nach Beginn der Meßperiode, d.h. nach Einsetzen des Meßimpulses, und dauert jeweils zwischen 5 und 10 ms. Vorzugsweise beginnt die Integration jeweils ca. 15 ms nach Einsetzen des Meßimpulses und erfolgt in einem Zeitraum von 5 ms.

Vorteilhaft werden beim erfindungsgemäßen Verfahren der Sensorelektrode vor dem ersten Meßpotential zwei weitere Potentialstufen aufgeprägt. Die erste dieser beiden Potentialstufen liegt dabei im Bereich zwischen -0,5 und -1,5 V, vorteilhaft im Bereich zwischen -0,8 und -1,3 V, und vorzugsweise bei ca. -1,0 V; die zweite Potentialstufe liegt etwa bei 0 V (mit einer Bandbreite von ca. ± 200 mV). Die Verweildauer bei der ersten dieser weiteren Potentialstufen, d.h. die Impulslänge, beträgt vorteilhaft 10 bis 50 ms. Bei der zweiten zusätzlichen Potentialstufe beträgt die Verweildauer, d.h. der zeitliche Vorlauf (vor dem ersten Meßpotential), vorteilhaft 10 bis 150 ms.

Der Potentialsprung vor den Meßpotentialen dient beim erfindungsgemäßen Verfahren zur Messung der Flußgeschwindigkeit des Blutes, d.h. des Elektrolyten. Durch einen Impuls in einem zeitlich definierten Abstand vor den Meßpotentialen erfolgt nämlich an der Sensorelektrode und in deren unmittelbarer Nähe eine Reduktion von Sauerstoff; die Messung ergibt dann ein niedrigeres Meßsignal als ohne diesen Vorimpuls. Diese Verminderung (des Meßsignals) ist ein Maß dafür, in welchem Umfang der Sauerstoff im Elektrolyten im Bereich vor der Elektrode nachtransportiert wird. Wird nun abwechselnd mit und ohne Vorimpuls gemessen, so erhält man sowohl eine Information über den Sauerstoffpartialdruck als auch eine Information über den Transport von Sauerstoff zur Elektrode. Dies stellt aber eine Information über die Flußgeschwindigkeit dar.

Beim erfindungsgemäßen Verfahren kann ferner vor der Sauerstoffmessung eine Bestimmung der Kapazität der Sensorelektrode erfolgen und somit das gemessene Integral, d.h. der Sauerstoffwert, auf die jeweilige Elektrode bzw. deren individuelle Eigenschaften, Fläche und Aktivität bezogen werden. Dadurch wird es ermöglicht, verschiedene Elektroden - ohne spezielle Eichung - mit einer Elektronik zu verwenden. Bei einer derartigen Vorgehensweise kann sich unter Umständen sogar eine Kalibrierung im Blut oder einer NaCl-Lösung mit fest eingestellten Sauerstoffpartialdrücken erübrigen.

Ein Sauerstoffsensor nach der Erfindung, d.h. eine Vorrichtung zur Durchführung des erfindungsgemäßen Verfahrens, weist eine Sensorelektrode, eine Gegenelektrode und eine Bezugselektrode auf; die Sensorelektrode besteht dabei wenigstens an der Oberfläche aus elektrokatalytisch inaktivem Kohlenstoff. Bei diesem Sauerstoffsensor sind ferner Mittel vorgesehen, um der Sensorelektrode das gewünschte Potentialprofil aufzuprägen, den Strom zu ermitteln und zu integrieren und die Sauerstoffkonzentration zu bestimmen.

Als Material für die Sensorelektrode (bzw. für deren Oberfläche) dient vorteilhaft ein elektrokatalytisch inaktiver Kohlenstoff aus der Gruppe Glaskohlenstoff, Pyrographit, gesputterter Kohlenstoff, gesputteter Graphit und amorpher wasserstoffhaltiger Kohlenstoff (a-C:H); vorzugsweise wird dabei Glaskohlenstoff eingesetzt. Die Gegenelektrode besteht vorteilhaft aus Platin oder auch aus Titan. Die Bezugselektrode ist vorzugsweise eine Ag/AgCl-Elektrode.

Für die Patientenüberwachung und ähnliche Zwecke gelangt der erfindungsgemäße Sauerstoffsensor vorteilhaft in Form eines Kathetersensors zum Einsatz. Ein derartiger Kathetersensor, der insbesondere zur Messung von Sauerstoff im Blut dient, weist folgende Dreielektrodenanordnung auf: die Sensorelektrode, d.h. die Arbeits- oder Meßelektrode, bildet die Katheterspitze, während die Bezugselektrode und die Gegenelektrode, jeweils mit Abstand voneinander, dahinter auf dem Katheter angeordnet sind. Der Abstand zwischen der Sensorelektrode und der davon isolierten Bezugselektrode beträgt dabei beispielsweise 1 bis 3 mm, der Abstand zwischen der Bezugselektrode und der Gegenelektrode 2 bis 5 mm. Von Vorteil ist es, wenn die Bezugs- und die Gegenelektrode hülsenförmig ausgestaltet sind.

Als eigentliches Kathetermaterial kommen insbesondere Polyurethan und Polyorganosiloxane, d.h. Silicone, in Betracht. Der Katheter selbst wird beispielsweise im Guß- oder Spritzgußverfahren hergestellt, wobei die Elektroden vorher kontaktiert werden und ein Anschlußstecker, der im allgemeinen dreipolig ist, mit eingegossen wird. Vorteilhaft kann noch vorgesehen werden, daß zusätzlich die Temperatur des Katheters gemessen wird, was mit einem Thermistor erfolgen kann; in diesem Fall wird dann ein fürfpoliger Stecker verwendet. Außerdem kann eine Messung des Druckes vorgesehen werden. Zu diesem Zweck wird dann im Katheter noch eine Kapillare angeordnet.

Anhand von Ausführungsbeispielen soll die Erfindung noch näher erläutert werden.

Die Länge eines Kathetersensors, der im Blutstrom angeordnet werden soll, beträgt beispielsweise 10 bis 12 cm, der Durchmesser 0,50 bis 0,75 mm. Die Sensorelektrode, die beispielsweise aus Glaskohlenstoff besteht, weist eine Oberfläche von ca.
1 mm² auf, bestehend aus einer Halbkugel und einem Zylindermantel. Die Bezugselektrode, insbesondere eine Ag/AgCl-Elektrode, weist eine Oberfläche von ca. 0,45 mm² auf, bestehend aus einem Zylindermantel (mit einer freien Länge von 1 mm). Die Gegenelektrode, die vorzugsweise aus Platin oder Titan besteht, weist eine Oberfläche von ca. 1,1 mm² auf, bestehend aus einem Zylindermantel (mit einer freien Länge von 2,5 mm). Der Isolationswiderstand zwischen den Elektroden beträgt ca. 1.10⁹Ω; der Zuleitungswiderstand der Sensorelektrode liegt unterhalb 10Ω.

Die Ansteuerschaltung des Kathetersensors besteht im allgemeinen aus einem Potentiostat, einem Spannungsgeber und einem Integrier- und Rechenglied. Im Betrieb gelten beispielsweise folgende Werte: Eingangswiderstand der Bezugselektrode: 1.10⁹Ω; Kapazität des Bezugselektrodeneingangs: 20 pF; maximaler Strom über die Gegenelektrode: 10 mA. Diese Werte können aber den jeweiligen Gegebenheiten angepaßt werden.

Die Begrenzung des Stromanstiegs und des maximalen Stromes ist einstellbar. Der Spannungsgeber liefert beispielsweise folgende Potentialstufen: -800 mV/20 ms, -1000 mV/20 ms, 0 mV/1960 ms; dabei gilt: Zeitauflösung: 1 ms; Spannung: 5 mV absolut, 1 mV reproduzierbar. Die Integration des Stromes in der ersten und der zweiten Potentialstufe erfolgt jeweils im Zeitraum von 15 bis 20 ms nach Beginn des Impulses; der zu erwartende Strom beträgt dabei etwa 10 bis 200 µA.

Der Abgriff des Stromes erfolgt als Spannungsabfall an einem Widerstand im Gegenelektrodenkreis mit einem Instrumentenverstärker; die Messung des Ausgangssignals bzw. die Weiterverarbeitung geschieht digital oder analog. In beiden Stufen beträgt die gemessene Ladung etwa 50 nAs bis 1 µAs. Die Differenzbildung der beiden Ladungen ergibt einen Wert, der ein Maß für die Sauerstoffkonzentration ist. Für die Meßgenauigkeit der Ladung gilt: Auflösung: 0,2 nAs; Meßbereich: 0 bis 2000 nAs.

Die Berechnung des Sauerstoffpartialdrucks (pO₂) aus der Ladung (Q) erfolgt nach:

pO₂ = A.Q + B (lineare Gleichung)

oder

pO₂ = A.Q² + B.Q + C (Parabel).

Die Bestimmung der Koeffizienten A, B und C erfolgt durch Messung der Elektrodenkapazität bei 1 kHz im Blut zu Beginn der Sauerstoffmessung, verbunden mit einer In-vitro-Eichung nach Herstellung der Elektrode.

## Patentansprüche

1. Verfahren zur elektrochemischen Bestimmung der Sauerstoffkonzentration, insbesondere in Körperflüssigkeiten, mittels eines eine Sensorelektrode aufweisenden Sauerstoffsensors, das folgende Schritte umfaßt:
- der Sensorelektrode wird ein Potentialprofil mit mehreren Potentialstufen aufgeprägt, wobei die erste Potentialstufe entsprechend einem 1. Meßpotential im Bereich zwischen -0,5 und -1,2 V, die zweite Potentialstufe entsprechend einem 2. Meßpotential im Bereich zwischen -0,5 und -1,3 V und die dritte Potentialstufe etwa bei 0 V liegt, jeweils bezogen auf eine Ag/AgCl-Referenzelektrode, und wobei zwischen den beiden Meßpotentialen eine Potentialdifferenz von ≧ 50 mV besteht und die Verweildauer bei den beiden Meßpotentialen, d.h. die Meßperiode, jeweils zwischen 10 und 50 ms beträgt
- der bei den beiden Meßpotentialen fließende Strom wird ermittelt und über die Zeit integriert, wobei die Integration jeweils etwa 5 bis 30 ms nach Beginn der Meßperiode einsetzt und jeweils zwischen 5 und 10 ms dauert,
- durch Differenzbildung der beiden Integrale wird die Sauerstoffkonzentration ermittelt.

2. Verfahren nach Anspruch 1, **dadurch gekennzeichnet**, daß die Verweildauer bei der dritten Potentialstufe zwischen 0,5 und 10 s beträgt.

3. Verfahren nach Anspruch 1 oder 2, **dadurch** **gekennzeichnet**, daß das erste Meßpotential im Bereich zwischen -0,75 und -1,15 V und/oder das zweite Meßpotential im Bereich zwischen -0,85 und -1,25 V liegt.

4. Verfahren nach einem der Ansprüche 1 bis 3, **dadurch gekennzeichnet**, daß der Sensorelektrode vor dem ersten Meßpotential zwei weitere Potentialstufen aufgeprägt werden, wobei die erste dieser beiden Potentialstufen im Bereich zwischen -0,5 und -1,5 V und die zweite dieser beiden Potentialstufen etwa bei 0 V liegt.

5. Verfahren nach Anspruch 4, **dadurch gekennzeichnet**, daß die Verweildauer bei der ersten der beiden weiteren Potentialstufen zwischen 10 und 50 ms und/oder bei der zweiten der beiden weiteren Potentialstufen zwischen 10 und 150 ms beträgt.

## Claims

1. A process for electrochemically determining oxygen concentration, in particular in body fluids, by means of an oxygen sensor having a sensor electrode, comprising the following steps:
- a potential profile having a plurality of potential stages is impressed on the sensor electrode, the first potential stage - corresponding to a first measuring potential - being in the range between -0.5 and -1.2 V, the second potential stage - corresponding to a second measuring potential - being in the range between -0.5 and -1.3 V, and the third potential stage being about 0 V, referred in each case to an Ag/AgCl reference electrode, there being a potential difference between the two measuring potentials of ≧50 mV and the length of stay at the two measuring potentials, i.e. the measurement period, being in each case between 10 and 50 ms,
- the current flowing at the two measuring potentials is determined and integrated with respect to time, the integration starting in each case about 5 to 30 ms after the start of the measurement period and lasting in each case between 5 and 10 ms, and
- the oxygen concentration is determined by taking the difference between the two integrals.

2. A process according to claim 1,characterised in that the length of stay at the third potential stage is between 0.5 and 10 s.

3. A process according to claim 1 or claim 2, characterised in that the first measuring potential is in the range between -0.75 and -1.15 V and/or the second measuring potential is in the range between -0.85 and -1.25 V.

4. A process according to any of claims 1 to 3, characterised in that two further potential stages are impressed on the sensor electrode prior to the first measuring potential, the first of these two potential stages being in the range between -0.5 and -1.5 V and the second of these two potential stages being at about 0 V.

5. A process according to claim 4, characterised in that the length of stay at the first of the two further potential stages is between 10 and 50 ms and/or at the second of the two further potential stages it is between 10 and 150 ms.

## Revendications

1. Procédé pour la détermination électrochimique de la concentration en oxygène, surtout dans les fluides corporels, au moyen d'un détecteur d'oxygène muni d'une électrode de détection, qui comprend les étapes suivantes :
· on impose un profil de potentiel à l'électrode de détection avec plusieurs niveaux de potentiel, où le premier niveau de potentiel - correspondant à un 1^{er} potentiel de mesure - se situe dans le domaine entre -0,5 et -1,2 V, le deuxième niveau de potentiel - correspondant à un 2^{e} potentiel de mesure - se situe dans le domaine entre -0,5 et -1,3 V et le troisième niveau de potentiel s'élève à environ 0 V, chaque fois par rapport à une électrode de référence Ag/AgCl, et où il existe une différence de potentiel entre ces deux potentiels de mesure de ≧ 50 mV et où le temps de séjour durant les deux potentiels de mesure, c'est-à-dire la période de mesure, se situe chaque fois entre 10 et 50 ms,
· le courant circulant lors des deux potentiels de mesure est déterminé et intégré par rapport au temps, où l'intégration commence chaque fois environ 5 à 30 ms après le début de la période de mesure et dure chaque fois entre 5 et 10 ms,
· la concentration en oxygène est déterminée par la différence des deux intégrales.

2. Procédé selon la Revendication 1, caractérisé en ce que le temps de séjour se situe entre 0,5 et 10 s lors du troisième niveau de potentiel.

3. Procédé selon la Revendication 1 ou 2, caractérisé en ce que le premier potentiel de mesure se situe dans le domaine entre -0,75 et -1,15 V et/ou le deuxième potentiel de mesure se situe dans le domaine entre -0,85 et -1,25 V.

4. Procédé selon l'une quelconque des Revendications 1 à 3, caractérisé en ce qu'on impose à l'électrode de détection, avant le premier potentiel de mesure, deux niveaux de potentiel supplémentaires, où le premier de ces deux niveaux de potentiel se situe dans le domaine entre -0,5 et -1,5 V et où le deuxième de ces deux niveaux de potentiel s'élève à environ 0 V.

5. Procédé selon la Revendication 4, caractérisé en ce que le temps de séjour se situe entre 10 et 50 ms lors du premier des deux niveaux de potentiel supplémentaires et/ou le temps de séjour se situe entre 10 et 150 ms lors du deuxième des deux niveaux de potentiel supplémentaires.
